# EUROPEAN PATENT SPECIFICATION

(11) **EP 2 594 203 B1**
(45) Date of publication and mention of the grant of the patent: **05.08.2015**
(21) Application number: 12180997.4
(22) Date of filing: 20.08.2012
(51) Int. Cl.: A61B 8/00, G06F 3/023, H01H 13/83, H04M 1/725

(54) **Ultrasound apparatus for displaying a plurality of key-sets and ultrasound diagnosis method using the ultrasound apparatus**
Ultraschallvorrichtung zum Anzeigen mehrerer Tastaturen und Ultraschalldiagnoseverfahren unter Verwendung der Ultraschallvorrichtung
Appareil à ultrasons permettant d'afficher plusieurs ensembles de claviers et procédé de diagnostic par ultrasons utilisant l'appareil à ultrasons

(30) Priority: 16.11.2011 KR 20110119783
(43) Date of publication of application: 22.05.2013
(73) Proprietor: Samsung Medison Co., Ltd., Hongcheon-gun, Gangwon-do 250-875 (KR)
(72) Inventor: Woo, Kyeong-gu, Gangwon-do (KR); Kang, Hak-il, Gangwon-do (KR); Jo, Jae-moon, Gangwon-do (KR)
(74) Representative: Schmid, Wolfgang

(56) References cited:
- EP-A1- 1 758 140
- WO-A1-2006/040697
- FR-A1- 2 863 725
- US-A1- 2002 173 721
- US-B1- 6 491 630
- US-B1- 6 685 637

## Description

### BACKGROUND OF THE INVENTION

### 1. Field of the Invention

The present invention relates to an ultrasound apparatus for selectively displaying one of various key-sets on a control panel by controlling beams emitted from a light source, and an ultrasound diagnosis method using the ultrasound apparatus.

### 2. Description of the Related Art

An ultrasound diagnosis apparatus delivers an ultrasound signal from a body surface of a target object to a predetermined part in the body, and then obtains an image of tomography or hematocele of a soft tissue by using information of the ultrasound signal reflected from a tissue in the body.

The ultrasound diagnosis apparatus is advantageous in that it is small, is inexpensive, and displays in real-time. Also, the ultrasound diagnosis apparatus is not harmful such as radioactivity and thus is safe, so that it is widely used together with an X-ray diagnosis apparatus, a computerized tomography (CT) scanner, a magnetic resonance image (MRI) apparatus, a nuclear medicine diagnosis apparatus, and the like.

In general, the ultrasound diagnosis apparatus has a plurality of buttons for controlling various functions. The plurality of buttons are mounted in the ultrasound diagnosis apparatus via a plurality of button print processes, such that a defect rate of the ultrasound diagnosis apparatus is high.

Also, since the buttons of the ultrasound diagnosis apparatus are printed in several languages for export, there are many difficulties in stock management.

Therefore, it is required to provide a system capable of decreasing the number of button print processes for the ultrasound diagnosis apparatus, and efficiently performing stock management with respect to ultrasound diagnosis apparatuses that are printed in several languages.

US 6,685,637 B1 discloses an ultrasonic diagnostic imaging system which enables the system operator to change the language of the graphical user interface without the need to restart the system. The software build for text, dialog boxes, symbols, icons, and other language-specific information contains this display information in multiple languages. By accessing a setup menu or other selection screen the user can change to one of the other languages stored on the system. This change can be implemented without the need to reboot the system.

EP 1 758 140 A1 describes an arrangement and a method for displaying at least a first and a second image. The arrangement comprises at least one light source adapted to generate light having at least a first and a second light characteristic and at least a first and a second image forming element arranged to display a first and a second image, respectively. The first image forming element is at least responsive to the light having the first light characteristic. The second image forming element is at least responsive to the light having the second light characteristic.

### SUMMARY OF THE INVENTION

The present invention provides an ultrasound apparatus according to claim 1.

The plurality of key-sets may be printed on the manipulation unit by a special ink that responds to at least one beam from among the plurality of beams.

The plurality of beams may be classified according to colors or frequency characteristics.

The plurality of key-sets corresponds to a plurality of image modes, respectively

The plurality of image modes may include an ultrasound image mode, and the ultrasound image mode may include at least one of a brightness mode (B mode), a color mode (C mode), a motion mode (M mode), a pulsed-wave (PW) mode, a continuous wave (CW) mode, a two-dimensional (2D) mode, and a three-dimensional (3D) mode.

The control unit contols the light source to emit at least one beam from among the plurality of beams to the control panel, according to an image mode selected by a user.

According to another aspect of the present invention, there is provided an ultrasound diagnosis method according to claim 6.

According to another aspect of the present invention, there is provided a computer-readable recording medium having recorded thereon a program for executing the ultrasound diagnosis method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram illustrating a structure of an ultrasound apparatus according to an embodiment of the present invention;
FIG. 2 illustrates a light source related to the embodiment of the present invention in FIG. 1;
FIG. 3 is a flowchart of an ultrasound diagnosis method, according to an embodiment not being part of the present invention;
FIG. 4 is a diagram illustrating a plurality of key-sets that correspond to a plurality of languages, which are related to an embodiment not being part of the present invention; and
FIG. 5 is a diagram illustrating a plurality of key-sets that correspond to a plurality of image modes, which are related to an embodiment of the present invention.

### DETAILED DESCRIPTION OF THE INVENTION

Terms or words used in the following description should not be construed as being limited to common or general meanings but should be construed as fully satisfying the invention defined by the claims.

In the following description, the applicant arbitrarily selects some terms or words, and in those cases, the detailed meaning are provided here.

Also, when a part "includes" or "comprises" an element, unless there is a particular description contrary thereto, the part can further include other elements, not excluding the other elements. In the following description, terms such as "unit" and "module" indicate a unit for processing at least one function or operation, wherein the unit and the block may be embodied as hardware or software or embodied by combining hardware and software.

In the following description, "ultrasound image" indicates an image with respect to a target object, which is obtained by using ultrasound. The target object may indicate a part of a human body. For example, the target object may include human organs such as the liver, the heart, the womb, the brain, the breast, the abdomen, and the like, an unborn child, or the like.

In the following description, "user" may indicate a medical expert including a doctor, a nurse, a clinical pathologist, a medical image expert, or the like but is not limited thereto.

Hereinafter, the present invention will be described in detail by explaining exemplary embodiments of the invention with reference to the attached drawings. In the following description, functions or constructions that are not related to the present invention are omitted, and like reference numerals in the drawings denote like elements.

Expressions such as "at least one of," when preceding a list of elements, modify the entire list of elements and do not modify the individual elements of the list.

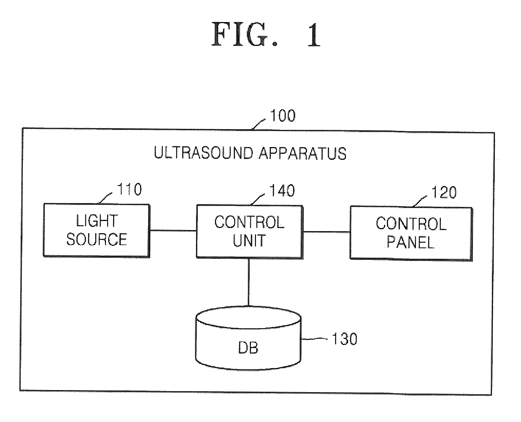

FIG. 1 is a block diagram illustrating a structure of an ultrasound apparatus 100 according to an embodiment of the present invention.

The ultrasound apparatus 100 indicates an apparatus that may obtain an ultrasound image with respect to a target object by using ultrasound and that may display the ultrasound image to a user.

The ultrasound apparatus 100 may be embodied in various forms. For example, the ultrasound apparatus 100 may be embodied as a fixed terminal or a mobile terminal. Examples of the mobile terminal may include a laptop computer, a person digital assistant (PDA), a tablet, or the like.

As illustrated in FIG. 1, the ultrasound apparatus 100 may include a light source 110, a control panel 120, a database (DB) 130, and a control unit 140. However, not every element of the ultrasound apparatus 100 is necessary. More elements or less elements than the aforementioned elements may embody the ultrasound apparatus 100.

The light source 110 may emit a plurality of beams. The plurality of beams may be classified according to colors or frequency characteristics. For example, the plurality of beams may include blue, red, green, yellow, purple, or the like.

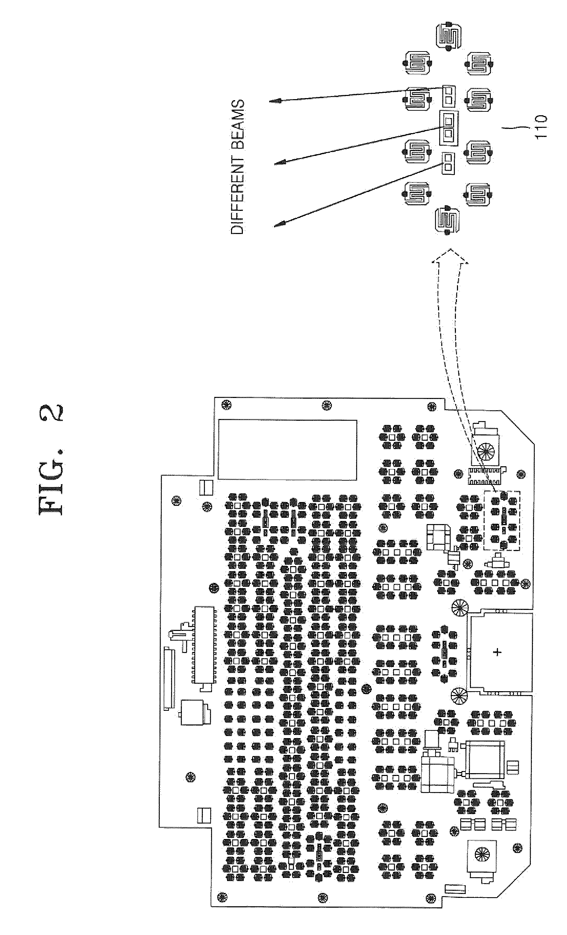

According to the present embodiment, as illustrated in FIG. 2, the light source 110 may be disposed in a predetermined region of a control panel (CP) board and may emit various types of beams. According to another embodiment, the light source 110 may separately exist outside of the ultrasound apparatus 100.

A plurality of key-sets may be printed on the control panel 120. In the present embodiment, the control panel 120 may be a button type control panel (i.e., a user input unit) and may be formed as a key pad.

The key-set indicates a group of input keys that are required to express a particular language or to control a particular image mode. The plurality of key-sets in the present embodiment may correspond to a plurality of languages (embodiment not included in the present invention), respectively, or a plurality of image modes, respectively.

For example, a key-set for the Korean language may include 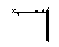 , 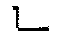 , 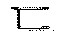 , 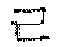 , 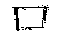 , 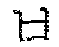 , 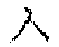 , 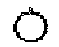 , 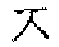 , 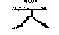 , 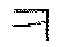 , 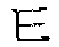 , 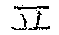 , 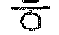 , 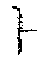 , 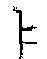 , 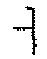 , 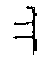 , 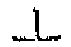 , 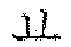 , 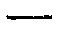 , any 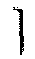 , a key-set for the English language may include a, b, c, d, e, f, g, h, i, j, k, l, m, n, o, p, q, r, s, t, u, v, w, x, y, and z, and a key-set for the Japanese language may include 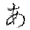 , 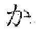 , 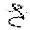 , 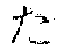 *,* 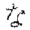 *,* 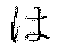 *,* 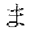 *,* 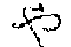 *,* 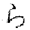 *,* 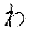 *,* 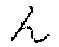 *,* 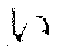 , 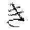 , 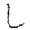 , 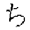 , 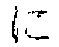 , 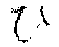 *,* 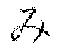 *,* 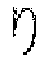 , 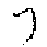 , 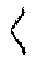 , 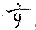 , 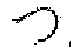 , 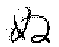 , 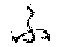 , 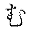 , 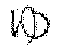 , 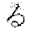 , 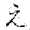 , 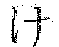 , 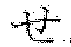 , 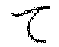 , 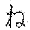 , 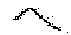 , 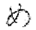 , 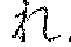 , 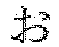 , 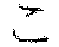 , 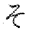 , 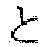 , 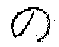 , 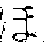 , 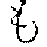 , 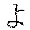 , 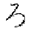 , and 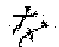 .

According to the present embodiment, the key-set corresponding to the particular image mode may be a key-set for functions or menus that are the most-frequently-used in all image modes.

Each of the plurality of key-sets may respond to at least one beam from among the plurality of beams emitted from the light source 110 and thus may be displayed on the control panel 120.

For example, if a beam emitted from the light source 110 is a blue beam, a key-set for the Korean language may respond, and if a beam emitted from the light source 110 is a red beam, a key-set for the English language may respond.

According to the present embodiment, the plurality of key-sets may be printed on the control panel 120 by a special ink. The special ink may allow the plurality of key-sets to respond to at least one beam.

That is, according to the present embodiment, special inks that respond to particular beams, respectively, are sprayed on the control panel 120 a plurality of times, so that the plurality of key-sets that are used for various languages or various modes may be selectively displayed on the control panel 120.

According to the present embodiment, in a case where a language or an image mode used in the ultrasound apparatus 100 is added, a key-set may be additionally printed on the control panel 120.

An image mode according to the present embodiment may include an ultrasound image mode. The ultrasound image mode may be at least one of a brightness mode (B mode), a color mode (C mode), a motion mode (M mode), a pulsed-wave (PW) mode, a continuous wave (CW) mode, a two-dimensional (2D) mode, and a three-dimensional (3D) mode. Alternatively, the ultrasound image mode may be a composite mode formed by combining at least two image modes.

The DB 130 may store information about a key-set. For example, the DB 130 may store information about the key-set corresponding to the particular language or the key-set corresponding to the particular image mode.

The DB 130 may also store information about languages or information about image modes, which may be used or set in the ultrasound apparatus 100. Also, the DB 130 may store information about keys that are frequently used by a user in the particular image mode.

The DB 130 may store a predetermined key-set and information about at least one beam corresponding to the predetermined key-set.

According to the present embodiment, the DB 130 may be included in the ultrasound apparatus 100 or may exist in the form of an external memory. Also, the DB 130 may be a storage server that is embodied on web.

The control unit 140 may control all operations of the light source 110, the control panel 120, and the DB 130. According to the present embodiment, the control unit 140 may control the light source 110 to emit at least one beam to the control panel 120.

The control unit 140 may receive selection information from an external source so as to select a predetermined language or a predetermined image mode. In this case, the control unit 140 may control the light source 110 to emit at least one beam from among the plurality of beams to the control panel 120 according to the selection information.

For example, in an embodiment not included in the present invention, when a user selects a language to be applied to the ultrasound apparatus 100, the control unit 140 may control a beam emitted from the light source 110 so as to allow a key-set, which corresponds to the selected language, to be displayed on the control panel 120.

According to an embodiment of the present invention, when the user selects at least one image mode from among a plurality of ultrasound image modes, the control unit 140 may control a color or a frequency of a beam emitted from the light source 110 so as to allow a key-set, which corresponds to the selected image mode, to be displayed on the control panel 120.

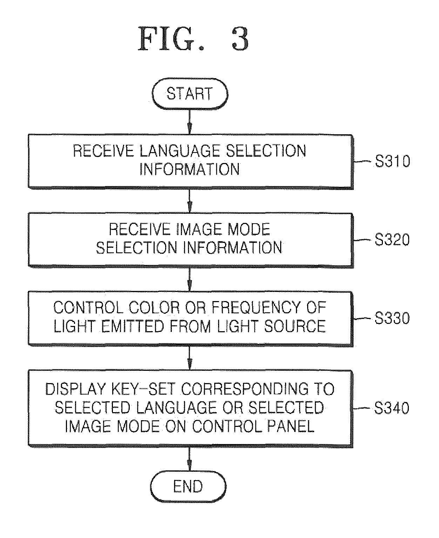

Hereinafter, an ultrasound diagnosis method of displaying a plurality of key-sets corresponding to a plurality of languages or a plurality of ultrasound image modes, by using each element of the ultrasound apparatus 100, is described in detail with reference to FIG. 3.

FIG. 3 is a flowchart of an ultrasound diagnosis method according to an embodiment not being part of the present invention.

As illustrated in FIG. 3, the method may include an operation of receiving language selection information from an external source so as to select a predetermined language (operation S310). That is, a user may select a language to be used in the ultrasound apparatus 100.

The language selection information may include selection information with respect to the Korean language, the English language, the Chinese language, the Japanese language, the French language, the German language, the Russian language, or the like but is not limited thereto.

The method may include an operation of receiving image mode selection information from an external source so as to select a predetermined image mode (operation S320). The user may select an image mode to be used in the ultrasound apparatus 100.

The image mode may include an ultrasound image mode, and the ultrasound image mode may include at least one of a B mode, a C mode, an M mode, a PW mode, a CW mode, a 2D mode, and a 3D mode.

Thus, according to the present embodiment, the image mode selection information may include selection information about at least one of the B mode, the C mode, the M mode, the PW mode, the CW mode, the 2D mode, and the 3D mode.

According to the present embodiment, the image mode selection information may include selection information about functions or menus that are frequently used in the ultrasound apparatus 100.

According to the an embodiment not being part of the present invention, operations S310 and S320 may be selectively performed. That is, the ultrasound apparatus 100 may receive only the language selection information or only the image mode selection information. Also, the ultrasound apparatus 100 may receive both of the language selection information and the image mode selection information.

For example, a user may select the Korean language as a language to be applied to the ultrasound apparatus 100. Also, the user may select the 3D mode so as to obtain a 3D ultrasound image. In this case, the user may select both the Korean language and a 3D image.

According to the present embodiment, the ultrasound apparatus 100 may control a color or a frequency of a beam emitted from the light source 110 so as to allow a key-set, which corresponds to the selection information, to be displayed on the control panel 120 (operation S330).

For example, in an embodiment not being part of the present invention, it is assumed that a key-set corresponding to the Korean language responds to a blue color, a key-set corresponding to the English language responds to a red color, and a key-set corresponding to the Japanese language responds to a green color.

If the user selects the Korean language as a language to be applied to the ultrasound apparatus 100, the control unit 140 may control the light source 110 to emit a blue beam to the control panel 120 from among a plurality of beams. Also, if the user selects the English language, the control unit 140 may control the light source 110 to emit a red beam.

In another embodiment, it is assumed that a key-set corresponding to the B mode responds to a purple color, a key-set corresponding to the C mode responds to a yellow color, and a key-set corresponding to the M mode responds to an orange color.

If the user selects the B mode, the control unit 140 may control the light source 110 to emit a purple beam from among the plurality of beams to the control panel 120. Also, if the user selects the C mode, the control unit 140 may control the light source 110 to emit a yellow beam.

According to the present embodiment, a key-set corresponding to the selected language may be displayed on the control panel 120 (operation S340).

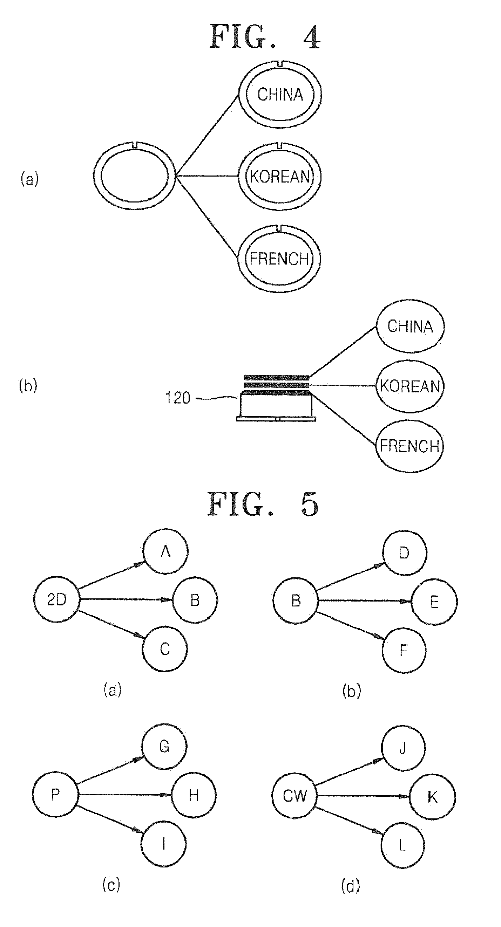

For example, as illustrated in FIG. 4, which is not part of the present invention, it is assumed that the Chinese language, the Korean language, and the French language may be printed on the control panel 120, and a user selects the Chinese language. In this case, the light source 110 may emit a beam to which a key-set corresponding to the Chinese language responds, and then the key-set corresponding to the Chinese language may be displayed on the control panel 120.

If the user changes a language of the ultrasound apparatus 100 from the Chinese language to the Korean language, the control unit 140 controls the light source 110 to emit a beam to the control panel 120, wherein a key-set corresponding to the Korean language may respond to the beam. Here, the key-set corresponding to the Korean language may be displayed on the control panel 120.

Thus, the ultrasound apparatus 100 may efficiently display a plurality of languages by using the least number of buttons.

According to an embodiment of the present invention, a key-set corresponding to a selected image mode may be displayed on the control panel 120 (operation S340).

For example, as illustrated in FIG. 5, when the 2D mode is selected, a key-set including A, B, and C corresponding to the 2D mode may be displayed on the control panel 120, when the B mode is selected, a key-set including D, E, and F corresponding to the B mode may be displayed on the control panel 120, and when the CW mode is selected, a key-set including J, K, and L corresponding to the CW mode may be displayed on the control panel 120.

Therefore, according to the other embodiment, the ultrasound apparatus 100 may efficiently display a plurality of key-sets, which correspond to a large number of image modes, respectively, by using a small number of buttons.

According to the one or more embodiments of the present invention, the ultrasound diagnosis method can also be embodied as computer-readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, etc. Also, the computer may include the control unit 140 of the ultrasound apparatus 100.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. An ultrasound apparatus (100) comprising:
a light source (110) for emitting a plurality of beams;
a control panel (120) on which a plurality of key-sets respectively
corresponding to a plurality of ultrasound image modes are printed, wherein each of the plurality of key-sets is configured to respond to at least one beam from among the plurality of beams and thus is displayed on the control panel (120);
and
a control unit (140) configured to receive selection information from an external source so as to select a predetermined ultrasound image mode and to control the light source (110) to emit at least one beam to the control panel (120) according to the selection information so as to allow a key-set, which corresponds to the selected ultrasound image mode, to be displayed on the control panel (120).

2. The ultrasound apparatus of claim 1, wherein the plurality of key-sets are printed on the control panel (120) by a special ink that responds to at least one beam from among the plurality of beams.

3. The ultrasound apparatus of claim 1, wherein the plurality of beams are classified according to colors or frequency characteristics.

4. The ultrasound apparatus of claim 1, wherein the ultrasound image mode comprises at least one of a brightness mode (B mode), a color mode (C mode), a motion mode (M mode), a pulsed-wave (PW) mode, a continuous wave (CW) mode, a two-dimensional (2D) mode, and a three-dimensional (3D) mode.

5. The ultrasound apparatus of claim 1, wherein the control unit (140) controls the light source (110) to emit at least one beam from among the plurality of beams to the control panel (120), according to an ultrasound image mode selected by a user.

6. An ultrasound diagnosis method comprising:
selecting at least one predetermined ultrasound image mode from among a plurality of ultrasound image modes according to a selection information received from an external source; and
displaying at least one key-set corresponding to the at least one ultrasound image mode on a control panel (120) by controlling a color or a frequency of a beam emitted from a light source (110),
the light source (110) being suitable for emitting a plurality of beams, wherein, on the control panel (120), a plurality of key-sets respectively corresponding to the plurality of ultrasound image modes are printed, each of the plurality of key-sets responding to at least one beam from among the plurality of beams.

7. The ultrasound diagnosis method of claim 6, wherein the ultrasound image mode comprises at least one of a brightness mode (B mode), a color mode (C mode), a motion mode (M mode), a pulsed-wave (PW) mode, a continuous wave (CW) mode, a two-dimensional (2D) mode, and a three-dimensional (3D) mode.

8. The ultrasound diagnosis method of claim 6, wherein the plurality of key-sets are printed on the control panel (120) by a special ink that responds to at least one beam from among a plurality of beams emitted from the light source (110).

9. A computer-readable recording medium having recorded thereon a program for executing the ultrasound diagnosis method of claim 6 on the ultrasound apparatus of claim 1.

## Patentansprüche

1. Ultraschallvorrichtung (100), welche Folgendes aufweist:
eine Lichtquelle (110) zum Emittieren einer Vielzahl von Strahlen;
ein Steuerpanel (120), auf welchem eine Vielzahl von Tastenanordnungen gedruckt sind, die jeweils einer Vielzahl von Ultraschallbildmodi entsprechen, wobei jede der Vielzahl von Tastenanordnungen dafür vorgesehen ist, auf wenigstens einen Strahl aus der Vielzahl von Strahlen zu reagieren und dadurch auf dem Steuerpanel (120) angezeigt wird; und
eine Steuereinheit (140), die dafür vorgesehen ist, Auswahlinformationen von einer externen Quelle zu empfangen, um einen vorbestimmten Ultraschallbildmodus auszuwählen und die Lichtquelle (110) zu steuern, um wenigstens einen Strahl gemäß der Auswahlinformationen zu dem Steuerpanel (120) zu emittieren, um es einer Tastenanordnung, welche dem ausgewählten Ultraschallbildmodus entspricht, zu erlauben, auf dem Steuerpanel (120) angezeigt zu werden.

2. Ultraschallvorrichtung nach Anspruch 1, wobei die Vielzahl von Tastenanordnungen auf dem Steuerpanel (120) mittels einer speziellen Tinte aufgedruckt ist, welche auf wenigstens einen Strahl unter der Vielzahl von Strahlen reagiert.

3. Ultraschallvorrichtung nach Anspruch 1, wobei die Vielzahl von Strahlen gemäß Farben oder Frequenzcharakteristika klassifiziert sind.

4. Ultraschallvorrichtung nach Anspruch 1, wobei der Ultraschallbildmodus wenigstens einen eines Helligkeitsmodus (B Modus), eines Farbmodus (C Modus), eines Bewegungsmodus (M Modus), eines gepulsten Wellen (PW)-Modus, eines kontinuierlichen Wellen (CW)-Modus, eines zweidimensionalen (2D)-Modus und eines dreidimensionalen (3D)-Modus aufweist.

5. Ultraschallvorrichtung nach Anspruch 1, wobei die Steuereinheit (140) die Lichtquelle (110) so steuert, dass sie wenigstens einen Strahl aus der Vielzahl von Strahlen gemäß einem von einem Benutzer ausgewählten Ultraschallbildmodus zu dem Steuerpanel (120) emittiert.

6. Ultraschalldiagnoseverfahren welches Folgendes aufweist:
Auswählen wenigstens eines vorbestimmten Ultraschallbildmodus aus einer Vielzahl von Ultraschallbildmodi gemäß einer von einer externen Quelle empfangenen Auswahlinformation; und
Anzeigen wenigstens einer Tastenanordnung, die dem wenigstens einen Ultraschallbildmodus entspricht, auf einem Steuerpanel (120) durch Steuern einer Farbe oder einer Frequenz eines von einer Lichtquelle (110) emittierten Strahls, wobei die Lichtquelle (110) dafür geeignet ist, eine Vielzahl von Strahlen zu emittieren,
wobei auf dem Steuerpanel (120) eine Vielzahl von Tastenanordnungen gedruckt ist, die jeweils der Vielzahl von Ultraschallbildmodi entspricht, wobei jede der Vielzahl von Tastenanordnungen auf wenigstens einen Strahl aus der Vielzahl von Strahlen reagiert.

7. Ultraschalldiagnoseverfahren nach Anspruch 6, wobei der Ultraschallbildmodus wenigstens einen eines Helligkeitsmodus (B Modus), eines Farbmodus (C Modus), eines Bewegungsmodus (M Modus), eines gepulsten Wellen (PW)-Modus, eines kontinuierlichen Wellen (CW)-Modus, eines zweidimensionalen (2D)-Modus und eines dreidimensionalen (3D)-Modus aufweist.

8. Ultraschalldiagnoseverfahren nach Anspruch 6, wobei die Vielzahl von Tastenanordnungen auf dem Steuerpanel (120) mittels einer speziellen Tinte aufgedruckt ist, welche auf wenigstens einen Strahl unter der Vielzahl von Strahlen, die von der Lichtquelle (110) emittiert werden, reagiert.

9. Computerlesbares Aufzeichnungsmedium, auf dem ein Programm zum Ausführen des Ultraschalldiagnoseverfahrens nach Anspruch 6 auf der Ultraschallvorrichtung nach Anspruch 1 aufgezeichnet ist.

## Revendications

1. Appareil ultrasonique (100) comportant :
une source lumineuse (110) pour émettre une pluralité de faisceaux ;
un panneau de contrôle (120) sur lequel sont imprimés une pluralité de repères clés, correspondant respectivement à une pluralité de modes d'images ultrasoniques ;
dans lequel chacun des repères clés est configuré pour répondre à au moins un faisceau de l'un de la pluralité de faisceaux et est ainsi affiché sur le panneau de contrôle (120) ;
et
une unité de commande (140) configurée pour recevoir une information sélective de la source extérieure afin de sélectionner un mode d'image ultrasonique prédéterminé pour commander la source de lumière (110) pour émettre au moins un faisceau vers le panneau de contrôle (120), correspondant à l'information sélectionnée, de manière à autoriser à un repère clé, qui correspond au mode de l'image ultrasonique sélectionnée, d'être affiché sur le panneau de contrôle (120).

2. Appareil ultrasonique selon la revendication 1, dans lequel la pluralité des repères clés est imprimée sur le panneau de contrôle (120) à l'aide d'une encre spéciale qui répond à au moins un faisceau parmi la pluralité de faisceaux.

3. Appareil ultrasonique selon la revendication 1, dans lequel la pluralité de faisceaux est classée en fonction des couleurs ou des caractéristiques de fréquences.

4. Appareil ultrasonique selon la revendication 1, dans lequel le mode de l'image ultrasonique comporte au moins un mode de brillance (Mode B), un mode de couleur (Mode C), un mode de mouvement (Mode M), un mode d'onde pulsée (PW), un mode d'onde continue (CW), un mode à deux dimensions (2D) et un mode à trois dimensions (3D).

5. Appareil ultrasonique selon la revendication 1, dans lequel l'unité de commande (140) commande la source de lumière (110) pour émettre au moins un faisceau parmi ladite pluralité de faisceaux vers le panneau de contrôle (120) correspondant à un mode d'image ultrasonique choisi par l'utilisateur.

6. Procédé de diagnostic ultrasonique comportant :
la sélection d'au moins un mode d'image ultrasonique prédéterminée parmi une pluralité de modes d'images ultrasoniques correspondant à une information sélectionnée provenant d'une source lumineuse ; et
l'affichage d'au moins un repère clé correspondant audit au moins un mode d'image ultrasonique sur le panneau de contrôle (120) en commandant une couleur ou une fréquence d'un faisceau émis par une source lumineuse (110),
une source de lumière (110) étant disponible pour émettre une pluralité de faisceaux,
dans lequel, sur le panneau de contrôle (120), une pluralité de repères clés correspondant respectivement à la pluralité de modes d'images ultrasoniques est imprimée, chacun de la pluralité de repères clés répondant à au moins un faisceau de la pluralité de faisceaux.

7. Procédé de diagnostic ultrasonique selon la revendication 6, dans lequel le mode d'image ultrasonique comprend au moins un mode de brillance (Mode B), un mode de couleur (Mode C), un mode de mouvement (Mode M), un mode d'onde pulsée (PW), un mode d'onde continue (CW), un mode à deux dimensions (2D) et un mode à trois dimensions (3D).

8. Procédé de diagnostic ultrasonique selon la revendication 6, dans lequel la pluralité des repères clés est imprimée sur le panneau de contrôle (120) à l'aide d'une encre spéciale qui répond à au moins un faisceau parmi la pluralité de faisceaux émis par la source de lumière (110).

9. Support lisible par un ordinateur sur lequel est enregistré un programme pour effectuer le procédé de diagnostic ultrasonique de la revendication 6 sur l'appareil selon la revendication 1.
